# FASCICULE DE BREVET EUROPEEN

(11) **EP 2 588 845 B1**
(45) Date de publication et mention de la délivrance du brevet: **04.05.2016**
(21) Numéro de dépôt: 11743272.4
(22) Date de dépôt: 30.06.2011
(51) Int. Cl.: G01N 11/10, G01N 33/543

(54) **PROCEDE DE DETECTION D'INTERACTIONS MOLECULAIRES**
VERFAHREN FÜR DEN NACHWEIS VON MOLEKULAREN INTERAKTIONEN
PROCESS OF DETECTION OF MOLECULAR INTERACTIONS

(30) Priorité: 19.08.2010 FR 1056678; 02.07.2010 FR 1002810
(43) Date de publication de la demande: 08.05.2013
(73) Titulaire: Biofilm Control, 63360 Saint-Beauzire (FR)
(72) Inventeur: BERNARDI, Thierry, 63170 Perignat Les Sarliève (FR); MAYER, Pascal, 63200 Marsat (FR); GROELLY, Jérôme, 63110 Beaumont (FR)
(74) Mandataire: Novagraaf Technologies
(86) Numéro de dépôt international: PCT/FR2011/051528
(87) Numéro de publication internationale: WO 2012/001312

(56) Documents cités:
- FR-A1- 2 866 706
- FR-A1- 2 883 296
- US-A- 4 935 147

## Description

### Domaine technique

La présente invention se rapporte à un procédé de détection d'interactions moléculaires. En particulier, la présente invention se rapporte à un procédé de détection d'interactions moléculaires entre au moins deux substances susceptibles d'interagir entre elles.

La présente invention trouve une application notamment dans le domaine de la recherche scientifique, dans le domaine médical, en particulier dans l'analyse d'échantillons biologiques.

Dans la description ci-dessous, les références entre parenthèses (Ref.) renvoient à la liste des références présentée à la fin du texte.

### Etat de la technique

Dans le domaine médical et analytique, il existe de nombreuses situations où il est intéressant, voire nécessaire de détecter des évènements d'interactions entre des molécules et/ou des objets comme des anticorps, des cellules, des bactéries, des virus et des macromolécules. Par exemple pour détecter une contamination bactérienne, un test antigène/ anticorps est fréquemment utilisé.

Pour déterminer le groupe sanguin d'une personne, un test d'affinité entre des anticorps et les globules rouges de la personne est fréquemment utilisé permettant ainsi de déterminer le groupe sanguin de ladite personne.

Il existe dans l'état de la technique des méthodes « simples » qui sont réalisables lorsque des substances à détecter sont présentes en grand nombre. Il peut s'agir par exemple d'une méthode comprenant un mélange de substances à détecter avec des substances d'affinité, c'est-à-dire des substances capables d'interagir avec les substances à détecter. L'interaction permet de former un précipité ou un agrégat visible à l'oeil nu et révélant le résultat. C'est de cette façon, par exemple, qu'il est possible de réaliser le typage de groupe sanguin, en ajoutant des anticorps à une goutte de sang et en observant la formation ou la non-formation d'un agrégat de globules rouges.

Il existe également des procédés qui permettent de mesurer les variations de viscosité d'un milieu qui se produisent lorsque des substances d'affinité s'assemblent dans ce milieu.

Par exemple, le document US 3 635 678 (Ref 1) décrit un procédé dans lequel une seule bille en acier de taille macroscopique (largement supérieure au micron) immergée dans le fluide est mise en suspension par un jeu d'aimants, le mouvement de la bille en suspension étant mesuré par un système optique. Quand la viscosité du milieu augmente l'amplitude du mouvement de la bille diminue au cours du temps, l'objectif est de déduire la vitesse de coagulation du sang.

Un inconvénient majeur de la technique décrite dans ce document est la complexité de sa mise en oeuvre car une bille doit être maintenue en suspension. D'autre part, la bille en mouvement, de part sa taille et sa masse telles que décrites dans le brevet, peut casser des interactions faibles à l'origine de la variation de viscosité et empêcher leur détection. La sensibilité de la méthode est faible et se limite à la détection de variations de viscosité relativement importantes. En outre, pour la lecture des résultats des systèmes complexes et des réactifs doivent être utilisés.

Un autre système est décrit dans le document WO 01/86255 (Ref 2) comportant un microscope avec lequel il est possible d'observer un mouvement d'une particule en suspension dans un liquide. La viscosité du liquide est déduite du déphasage de la seconde harmonique du signal obtenu à partir de l'observation de la particule en suspension dans un liquide avec le microscope.

Une autre variante de cette approche est décrite dans le document EP 1 544 596 (Ref 3), qui comprend l'oscillation d'une particule magnétisable dans un champ magnétique pour générer un signal et donc obtenir un résultat.

Le document FR 2 866 706 décrit un procédé qui permet d'étudier le développement d'un biofilm dans un milieu de culture non homogène. Selon ce document, on détermine la viscosité du milieu en immergeant au moins une particule magnétique dans la culture, en soumettant la culture à un champs magnétique et en détectant le degré de liberté de mouvement de la particule magnétique.

Un des inconvénients majeur de ces méthodes est la complexité de leur mise en oeuvre et du suivit de l'oscillation d'une particule actionnée par une force appliquée de façon périodique et contrôlée. L'instrumentation complexe, couteuse et difficile à paramétrer d'une part pour mettre en oscillation et maintenir l'oscillation de la particule, et d'autre part pour observer le mouvement périodique de la particule.

En outre ces méthodes permettent uniquement de mesurer des modifications de viscosité qui doivent avoir lieu dans la zone d'oscillation de la particule en suspension. En outre les modifications de viscosité doivent être importantes pour être détectées. Ainsi, il est nécessaire de disposer de solutions qui contiennent un grand nombre de substances d'affinité. Ces procédés sont donc peu sensibles et ne permettent pas d'atteindre une précision satisfaisante pour des méthodes utilisant, par exemple des anticorps, ou d'autres substances d'affinités dirigées spécifiquement à détecter ou doser.

Un autre procédé couramment utilisé pour détecter des réactions d'affinités est la technique ELISA et ses nombreuses déclinaisons. Elle consiste à :
- attacher sur un support un premier anticorps présentant une affinité avec une substance,
- mettre en contact pendant une durée déterminée la substance à détecter avec le premier anticorps attaché sur son support,
- retirer par rinçage la substance qui n'aurait pas réagit avec l'anticorps,
- mettre en contact la substance liée à l'anticorps attachés à la surface et un second anticorps présentant une affinité avec ladite substance,
- retirer par rinçage les anticorps qui n'auraient pas réagit avec la substance, et
- détecter la présence du second anticorps lié à la substance liée au premier anticorps lié au support.

Le plus souvent, le second anticorps est lié à un marqueur qui est détectable directement par une méthode physique, par exemple un marqueur fluorescent ou magnétique, ou à un marqueur lié à une enzyme, par exemple la phosphatase alcaline ou la peroxydase de radis noir, afin de détecter le produit de la réaction catalysée par l'enzyme à partir d'un substrat adapté.

Ce procédé présente plusieurs inconvénients, par exemple il nécessite nombreux lavages contrôlés diminuant sa sensibilité. Il comprend également de nombreuses étapes de manipulations par l'homme du métier; la réalisation d'au moins deux réactions d'affinités ; le marquage obligatoire du second anticorps avec, au moins un marqueur sensible et sophistiqué, l'utilisation d'un dispositif complexe et difficile à paramétrer pour détecter et quantifier le signal émit par le marqueur. Ce procédé est donc long, couteux, et nécessite une instrumentation complexe pour sa mise en oeuvre.

D'autres méthodes ont été développées pour détecter une réaction d'affinité ne nécessitant qu'un seul anticorps, par exemple des techniques basées sur la résonnance de plasmon de surface (« plasmon surface resonance »), des balances piézo-électriques, voire une simple observation par microscope pour certaines substances le permettant. Pour ce faire, l'anticorps est fixé sur un support adapté à la technique de détection, la substance est mise en contact pendant un temps donné avec l'anticorps fixé sur le support, puis la lecture est réalisée directement ou après un rinçage pour éliminer les substances n'ayant pas réagit par affinité avec les anticorps.

Ces méthodes présentent de nombreux inconvénients, par exemple l'instrument nécessaire pour réaliser la lecture est sophistiqué et couteux. En outre elles nécessitent des supports particuliers pour la fixation desdits anticorps. Par ailleurs, elles nécessitent un grand nombre d'étapes et sont dépendantes de la qualité et de la quantité d'anticorps fixés.

Dans les méthodes précitées, il est donc essentiel d'attacher l'anticorps ou toute autre substance d'affinité sur un support. Des surfaces d'affinité sont connues dans l'état de la technique. De telles surfaces peuvent être obtenues, par exemple par « moulage moléculaire ».

Il est également connu que l'on peut mettre en oeuvre les procédés précités en fixant de manière non spécifique la substance que l'on veut détecter sur un support. La substance ainsi fixée sera mise en contact avec un anticorps ou tout autre substance d'affinité adaptée, marqué ou non-marqué selon le procédé de détection, puis éventuellement de rincer avant de procéder à la lecture.

Toutefois, ces variantes présentent de nombreux inconvénients. En particulier, il est nécessaire de lier la substance à détecter sur le support, ce qui n'est pas possible de façon universelle ni spécifique. En outre, cette fixation peut modifier la structure de la substance fixée. Cette modification de structure peut altérer la sensibilité et/ou la spécificité de détection, en particulier lorsque des anticorps sont utilisés. En outre, la fixation peut conduire à l'obtention de résultats faux négatifs et/ou faux positifs. En outre, la fixation peut être différente d'une mise en oeuvre à une autre, les résultats ainsi obtenu peuvent donc être non reproductibles. De plus, pour la détection nécessite des dispositifs complexes et couteux.

Il existe donc un réel besoin de trouver un procédé de détection d'interactions moléculaires palliant ces défauts, inconvénients et obstacles de l'art antérieur, en particulier d'un procédé permettant d'améliorer la sensibilité de détection des interactions moléculaire, de réduire les coûts de mise en oeuvre, un procédé dont la mise en oeuvre est simple et qui permet d'obtenir des résultats rapides, fiable et reproductibles.

### Description de l'invention

La présente invention a précisément pour but de répondre aux nombreux besoins et inconvénients précités de l'art antérieur en fournissant un procédé de détection d'interactions moléculaire.

En particulier la présente invention a pour objet un procédé de détection d'interactions dans une solution comprenant les étapes suivantes:
a. introduire dans une solution au moins une première substance et de préférence au moins une seconde substance susceptible d'interagir avec ladite première substance,
b. introduire dans la solution obtenue à l'étape a) de 2 à 10 000 000 particules magnétiques ou magnétisables lesdites particules reposant sur une surface immergée dans ladite solution, lesdites particules ayant une taille comprise de 10 nm à 100 µm,
c. déterminer l'interaction desdites substances par application d'un champ électrique, magnétique ou électromagnétique destiné à mettre en mouvement lesdites particules, l'interaction entre lesdites substances étant détectée lorsque la mobilité desdites particules sur ladite surface est modifié.

Selon l'invention, par mobilité on entend le mouvement des particules par application et/ou sous l'effet du champ électrique, magnétique ou électromagnétique. Cette mobilité peut être définie, par exemple à partir du rapport entre la vitesse des particules à un point de l'espace donné et l'intensité du gradient du carré du champ magnétique à cet endroit de l'espace donné, par exemple à partir du rapport entre la vitesse des particules à un point de l'espace donné et l'intensité du gradient du potentiel électrique à cet endroit de l'espace donné.

Selon l'invention, la modification de la mobilité peut être choisie parmi un freinage, un ralentissement, une modification de la trajectoire, une accélération ou un arrêt desdites particules.

Ainsi, selon l'invention, l'application du champ électrique, magnétique ou électromagnétique peut provoquer un rassemblement, non rassemblement ou une dispersion desdites particules.

Selon l'invention, par interaction de substances on entend, par exemple une interaction moléculaire par exemple des interactions de type liaison hydrogène, ionique, van der Waals, une interaction biologique par exemple des interactions de reconnaissance de motifs tridimensionnels spécifiques de type hormone-récepteur, anticorps-antigènes, une interaction électrostatique, une interaction magnétique, un gradient de concentration d'ions ou de molécules, en d'autres termes, tout gradient de potentiel, de concentration moléculaire ou ionique qui trouve son origine au niveau des substances et qui tend à les faire se rapprocher ou se repousser, par exemple via des liaisons hydrogènes, des interactions de van der Waals, des liaisons hydrophobes, des liaisons covalentes, des liaisons ioniques, par exemple via des mouvement de chimiotaxie. Il peut s'agir par exemple d'interaction antigènes-anticorps, enzymes-substrats, récepteurs-ligands, molécules-cellules, cellules-vecteurs, cellules-virus, cellules eucaryotes-cellules procaryotes, cellules eucaryotes-cellules eucaryotes, cellules procaryotes-cellules procaryotes

La solution susceptible d'être utilisée dans la présente invention peut être une solution liquide ou un gaz. La solution peut être toute solution connue de l'homme du métier. Il peut s'agir par exemple un milieu de culture, par exemple un milieu de culture de cellules eucaryotes et/ou procaryotes, un milieu tampon, par exemple tout milieu tampon connue de l'homme du métier, par exemple un milieu tampon disponible dans le commerce, par exemple du tampon phosphate salin (PBS), un échantillon biologique, par exemple un échantillon de sang, de plasma, d'urine, de liquide céphalorachidien, une solution saline, par exemple une solution physiologique, un milieu de culture, par exemple de l'infusion de coeurs et de cerveaux disponible dans le commerce, d'un solvant, par exemple de l'acétone, du diméthylsulfoxyde, de l'éthanol, du méthanol, du propanol, de l'acétonitrile, l'acétate d'éthyle, l'éther, du phénol, du chloroforme, du tétrahydrofurane, du difluoroéthylène, et/ou un hydrocarbure, par exemple de l'hexane, du cyclo-hexane, du benzène, de l'octane, du décane, du pétrole, de l'essence, du gasoil.

Selon l'invention, le gaz peut être par exemple de l'air, de l'oxygène, de l'azote, du néon, de l'argon, du gaz-carbonique, du méthane, de l'ozone.

Selon l'invention une solution liquide peut avoir une masse volumique de 0,1 à 4 kg/l, de 0,3 à 3 kg/l ; une solution gazeuse peut avoir une densité de 10⁻¹⁵ kg/m³ à 1000 kg/m³, de 10⁻¹⁰ à 30 kg/m³, de 10⁻⁵ à 3 kg /m³.

L'homme du métier de part ces connaissances générales saura aisément déterminer la masse volumique d'une solution. Par exemple la mesure de la masse volumique de la solution peut être réalisée par exemple par mesure du ratio de la masse sur le volume, par exemple en pesant une solution de volume connue.

Selon l'invention, la solution peut être préalablement traitée, par exemple, la solution peut être purifiée, diluée, concentrée.

Selon l'invention, la solution peut être purifiée par tout procédé connu de l'homme du métier, par exemple par dialyse, par filtration, par ultrafiltration, par clarification et par centrifugation. Par exemple, le procédé de filtration peut comprendre le passage de la solution sur un tamis avec des pores de 0,2 à 100 µm, le procédé d'ultrafiltration peut comprendre, par exemple une centrifugation à une vitesse de 1 à 3000 tours par minute pendant un temps de 0,1 à 30 minutes, le procédé de dialyse peut être, par exemple un procédé comprenant une étape de dépôt de la solution sur une membrane de dialyse, par exemple à seuil de coupure de 500 Da, ladite membrane flottant sur de l'eau distillée contenue dans un récipient. Le procédé de clarification peut être, par exemple un procédé comprenant l'ajout dans la solution de 0.1% (poids/poids) d'albumine de sérum de bovin.

Selon l'invention, la purification de la solution peut permettre, avantageusement, d'éliminer de la solution tout contaminant et/ou molécules susceptibles d'altérer la détection de l'interaction moléculaire, par exemple la purification peut permettre d'éliminer indépendamment des bactéries, des virus, des protéines, des molécules chimiques, des sels, des grains de matières, des agrégats de molécules. Bien entendu, l'homme du métier de part ces connaissances générales saura adapter le procédé de purification en fonction de la solution

Selon l'invention, la solution peut être également diluée, par exemple tout procédé connu de l'homme du métier, par exemple par dilution sérielle. La dilution peut être réalisée avec tout diluant connu de l'homme du métier. Il peut s'agir par exemple d'une solution tampon, par exemple du tampon phosphate salin, une solution saline, par exemple du sérum physiologique, de l'éthanol, du DMSO, de l'acétone, de l'hexane et/ou tout solvant, hydrocarbure ou solution décrite précédemment.

La solution peut être diluée, par exemple d'un facteur de 2 à 20000, de 5 à 500, de 5 à 50.

La dilution de la solution peut permettre, avantageusement, de modifier la concentration des composants présents dans la solution, par exemple, en diminuer la concentration, par exemple la dilution peut permettre de diminuer la concentration protéique. La dilution peut permettre ainsi de diminuer la concentration d'éventuels composés interférents et ainsi avantageusement d'améliorer la spécificité et/ou la sensibilité du procédé de l'invention.

Selon l'invention, la solution peut être également concentrée, par exemple par tout procédé connu de l'homme du métier, par exemple par ultracentrifugation, par ultrafiltration, par évaporation, par lyophilisation

Selon l'invention, la purification, dilution et/ou concentration de ladite solution peut avantageusement permettre d'ajuster la masse volumique de ladite solution.

L'ajustement de la masse volumique de la solution permet avantageusement d'améliorer la spécificité et/ou la sensibilité du procédé de l'invention, notamment en augmentant, en diminuant ou en annulant l'effet de la force de pesanteur qui pousse les particules vers la surface.

Selon l'invention, le volume de la solution utilisée dans le procédé peut être, par exemple de 0.3 µl à 100 ml, de 3µl à 10ml, de 30 µl à 1 ml.

Selon l'invention, la solution peut être susceptible de moduler l'interaction entre ladite première et ladite seconde substance. Par exemple, la solution peut augmenter ou diminuer l'interaction entre lesdites substances.

Selon l'invention, la solution peut comprendre avantageusement un composé qui augmente ou diminue l'interaction entre lesdites première et seconde substances. Le composé peut être ajouté, par exemple, préalablement à la mise en oeuvre du procédé de l'invention dans la solution. Le composé peut être par exemple des molécules chimiques, des sels, des ions, des polymères des macromolécules, des colloïdes, des micro-particules, par exemple des acides et des bases qui modifient le pH de la solution, par exemple du NaCl qui modifie la force ionique de la solution, par exemple du polyéthylène-glycol qui peut par exemple augmenter l'affinité desdites substances.

La présente invention permet avantageusement de déterminer si ladite solution module effectivement l'interaction par comparaison des résultats obtenus par le procédé de l'invention dans lequel les substances sont identiques avec des solutions différentes.

Selon l'invention la première substance peut être choisie dans le groupe comprenant des cellules eucaryotes, des cellules procaryotes, des membranes, des virus, des prions, des mitochondries, des chloroplastes, des vésicules, des liposomes, des constituants cellulaires, des flagelles, des protéines, des lipo-protéines, des glyco-protéines, des anticorps, des acides nucléiques, des complexes lipidiques, des colloïdes, des macromolécules, des micro-particules, des nano-particules, des antigènes, des hormones, des ligands de protéines et des molécules chimiques.

Selon l'invention, les cellules eucaryotes susceptibles d'être utilisées dans la présente invention peuvent être par exemple des cellules eucaryotes animales, par exemples des cellules du sang, par exemple des leucocytes, par exemple des granulocytes, des polynucléaires neutrophiles ; des polynucléaires éosinophiles ; des polynucléaires basophiles ; des lymphocytes B, des lymphocytes T, des lymphocytes NK, des monocytes, des érythrocytes, des thrombocytes. Il peut s'agir également de cellules eucaryotes végétales, par exemple des cellules de l'épiderme végétal, du xylème, du phloème, du parenchyme, du collenchyme, du sclérenchyme. Il peut s'agir également de champignons, de levures. Il peut s'agir par exemple *Candida, Cryptococcus, Malassezia, Pityrosporum, Pneumocystis, Epidermo-phyton, Microsporum, Trichophyton.* Il peut s'agir également de protozoaires, par exemple *Entamoeba histolytica, Acanthamoeba castellanii, Naegleria fowleri.*

Selon l'invention, les cellules procaryotes peuvent être, par exemple toute bactérie connu de l'homme du métier. Les bactéries susceptibles d'être utilisées dans la présente invention sont par exemple les bactéries comprises dans le groupe, sans être limité à celui-ci, constitué de Acetobacter aurantius, Actinobacillus actinomycetemcomitans, Agrobacterium tumefaciens, Azorhizobium caulinodans, Azotobacter vinelandii, Bacillus anthracis, Bacillus brevis, Bacillus cereus, Bacillus fusiformis, Bacillus licheniformis, Bacillus megaterium, Bacillus stearothermophilus, Bacillus subtilis, Bacteroides gingivalis, Bacteroides melaninogenicus, Bartonella henselae, Bartonella quintana, Bordetella bronchiseptica, Bordetella pertussis, Borrelia burgdorferi, Branhamella catarrhalis, Brucella abortus, Brucella melitensis, Brucella suis, Burkholderia mallei, Burkholderia pseudomallei Calymmatobacterium granulomatis, Campylobacter coli, Campylobacter jejuni, Campylobacter pylori, Chlamydia pneumoniae, Chlamydia psittaci, Chlamydia trachomatis, Chlamydophila pneumoniae, Chlamydophila psittaci, Clostridium botulinum, Clostridium difficile, Clostridium perfringens, Clostridium tetani, Clostridium welchii, Corynebacterium diphtheriae, Corynebacterium fusiforme, Coxiella burnetii Ehrlichia chaffeensis, Enterococcus avium, Enterococcus durans, Enterococcus faecalis, Enterococcus faecium, Enterococcus galllinarum, Enterococcus maloratus, Escherichia coli Francisella tularensis, Fusobacterium nucleatum Gardnerella vaginalis Haemophilus ducreyi, Haemophilus influenzae, Haemophilus parainfluenzae, Haemophilus pertussis, Haemophilus vaginalis, Helicobacter pylori Klebsiella pneumoniae, Klebseilla rhinoscleromatis-klebsiella oxytoca Lactobacillus acidophilus, Lactobacillus casei, Lactococcus lactis, Legionella pneumophila, Methanobacterium extroquens, Microbacterium multiforme, Micrococcus luteus, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis , Mycoplasma fermentans, Mycoplasma genitalium, Mycoplasma hominis, Mycoplasma pneumoniae Neisseria gonorrhoeae, Neisseria meningitidis, Nocardia asteroides Pasteurella multocida, Pasteurella tularensis, Porphyromonas gingivalis, Pseudomonas aeruginosa, Pseudomonas maltophilia, Rhizobium radiobacter, Rickettsia prowazekii, Rickettsia mooseri, Rickettsia psittaci, Rickettsia quintana, Rickettsia rickettsii, Rickettsia trachomae, Rochalimaea henselae, Rochalimaea quintana, Rothia dentocariosa, Salmonella enteritidis, Salmonella typhi, Salmonella typhimurium, Serratia marcescens, Shigella dysenteriae, Staphylococcus aureus, Staphylococcus epidermidis, Streptococcus agalactiae, Streptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis, Streptococcus sobrinus, Treponema pallidum, Vibrio cholerae, Vibrio comma, Vibrio parahemolyticus, Vibrio vulnificus, Xanthomonas maltophilia Yersinia enterocolitica,Yersinia pestis et Yersinia pseudotuberculosis, etc.

Les membranes susceptibles d'être utilisées dans la présente invention peuvent être tout fragment de membranes de cellules eucaryotes, procaryotes, par exemple de cellules procaryotes ou eucaryotes précités, tout fragment de membrane cellulaire et/ou ou de compartiment cellulaire, par exemple de mitochondries, de chloroplastes, d'endoplastes, de reticulum endoplasmique.

Les protéines susceptibles d'être utilisées dans la présente invention peuvent être des protéines plasmatiques, des protéines cellulaires, des protéines bactériennes. Par exemple les protéines peuvent être des hormones, par exemple la progestérone, la vasopressine, l'hormone thyréotrope, l'hormone lutéinisante (LH), l'hormone de stimulation de la tyroïde (TSH), l'hormone de croissance (GH), du Facteur de croissance épidermal (EGF), de l'insuline, de l'ocytocine. Il peut s'agir par exemple de canaux, par exemple de canaux calciques, par exemple les canaux décrits dans Catterall WA. (2000) Structure and regulation of voltage-gated Ca²⁺ channels. Annu. Rev. Cell. Dev. Biol. 16:521-55 (Ref 4), de canaux potassiques, par exemple les canaux décrits dans Dick GM et Tune JD, Role of potassium channels in coronary vasodilation, Exp Biol Med (Maywood). 2010 Jan;235(1):10-22.(Ref 6). Il peut s'agir également de peptides impliquées dans le Complexe Majeur d'Histocompatibilité (CMH), par exemple le CMH I et/ou le CMH II. Il peut s'agir également de récepteurs, par exemple des récepteurs nucléaires, intracellulaire, membranaire, transmembranaire, couplés aux protéines G, de récepteurs de l'acétylcholine, de récepteur à la FSH, récepteur de la testostérone, par exemple les récepteur décrits dans Mostallino MC et collaborateurs, Plasticity and function of extrasynaptic GABA(A) receptors during pregnancy and after delivery, Psychoneuroendocrinology. 2009 Dec;34 Suppl 1:S74-83 (Ref 7). Il peut s'agir également d'enzymes, par exemple des enzymes bactériennes, par exemple des enzymes de restriction, par exemple Hindlll, Eco RI, BamHI, Mstll, Taql, NotI, Hinfl, AluI, BglIII, Haell, Hhal, Pstl, Smal, des enzymes impliquées dans la signalisation cellulaire, par exemple des protéine kinases, des enzymes impliquées dans la biosynthèse, par exemple la biosynthèses des acides gras, des phosphorylases, des déshydrogénases, par exemple la glucose déshydrogénase, des enzymes impliquées dans le métabolisme général, par exemple l'aldéhyde déshydrogénase, l'alpha-amylase, la L-gulonolactone oxidase, la rhodopsine, le cytochrome B, le cytochrome C. Il peut s'agir également de protéines de structure, pare exemple l'actine, la myosine, la peptine, l'albumine, le collagène, l'histone H4.

Les anticorps susceptibles d'être utilisés dans la présente invention peuvent être tout anticorps connu de l'homme du métier. Par exemple, il peut s'agir de tout anticorps disponible dans le commerce, par exemple des IgG, IgA, IgM, IgE et IgD, les anticorps peuvent être par exemple des anticorps dirigés contre une des protéines précitées, des anticorps dirigés contre d'autre anticorps, par exemple des anticorps de lapin dirigés contre des anticorps humains, par exemple des anticorps dirigés spécifiquement contre des anticorps dirigés contre le récepteur de la FSH. Il peut s'agir également d'anticorps obtenus après immunisation d'individu, par exemple selon le procédé décrit dans le document Biologie cellulaire et moléculaire, Concepts et expériences, 2^{nd} édition 2004, Gerald Karp (Ref 5). Il peut s'agir par exemple anticorps produits par un humain ou un animal en réponse à une infection, par exemple par un virus, par une bactérie, par un prion, par un parasite, par un protozoaire, mais aussi en réponse à une maladie, par exemple un cancer, une maladie auto-immune comme par exemple la maladie de Basedow, la sclérose en plaque, mais aussi en réponse à une intoxication, à un empoisonnement, à une contamination, à un dopage, à une ingestion, à une inhalation et à une injection, par exemple par un pesticide, par un poison, par un insecticide, par un herbicide, par un agent allergisant, mais aussi en réponse à une greffe, par exemple de moelle osseuse, d'organe comme par exemple un rein, un poumon, un foie, de membre comme par exemple de main, de jambe, de pied, mais aussi en réponse à une implantation d'organe artificiel, de chambre implantable, de pacemaker, de coeur artificiel, de hanche artificielle.

Les molécules chimiques susceptibles d'être utilisées dans le procédé de l'invention peuvent être toute molécule chimique connue de l'homme du métier. Il peut s'agir par exemple d'un réactif de coagulation, d'un réactif décrit dans le document, d'une hormone, par exemple des hormones stéroïdiennes, par exemple le cortisol, l'aldostérone, la progestérone, la déhydroépiandrostérone (DHEA), le sulfate de déhydroépiandrostérone (DHEAS), l'estradiol, l'androstènedione, la dihydrotestostérone (DHT), l'estrone, l'estriol, la testostérone, par exemple la thyroxine, par exemple des hormones peptidique, par exemple l'érythropoïétine (EPO), le glucagon, la somatostatine, le peptide natriurétique atrial (ANP), la corticotrophine (ACTH).

Selon l'invention, la première substance peut être présente dans la solution, dans ce cas, la première substance n'a pas besoin d'être introduite dans la solution.

Selon l'invention, ladite première substance peut être préalablement fixée sur la surface immergée. Le procédé de fixation de la première substance susceptible d'être utilisé dans la présente invention peut être tout procédé connu de l'homme du métier. Bien entendu, l'homme du métier de part ces connaissances générales saura choisir le procédé de fixation en fonction de la première substance.

Selon l'invention, lorsque la première substance est fixée, l'interaction moléculaire avec ladite seconde substance modifie la surface immergée et par exemple peut densifier cette surface sur laquelle repose ladite particule freinant ainsi le déplacement de ladite particule lorsque le champ électrique, magnétique ou électromagnétique est appliqué révélant ainsi l'interaction.

Selon l'invention, la seconde substance peut être toute substance définie ci-dessus susceptible d'interagir avec la première substance.

Selon l'invention, la masse volumique de la première et/ou de la seconde substance peut être supérieure ou inférieure à celle de la solution, de préférence supérieure à celle de la solution. Avantageusement, la supériorité de masse volumique de ladite première et/ou seconde substance permet une localisation de ladite première et/ou seconde substance à proximité du fond du contenant.

Selon un autre mode de réalisation de l'invention, le procédé de l'invention peut être mis en oeuvre avec une solution contenant préalablement ladite au moins une première, ladite au moins une seconde substance. Dans ce mode de réalisation, le procédé peut ne pas comprendre l'étape a) et peut comprendre uniquement les étapes b) et c) précitées.

Selon l'invention, le procédé peut comprendre à la place de l'étape a)
une étape préalable a') de fixation de ladite première substance sur une surface d'un contenant,
a") l'introduction d'une solution dans ledit contenant, et
a"') l'introduction d'au moins une seconde substance susceptible d'interagir avec ladite première substance.

Selon l'invention, le procédé de l'invention peut être mis en oeuvre avec une pluralité de particules, par exemple avec au moins 2 particules, avec par exemple de 2 à 10 000 000, de 1000 à 1 000 000, de 10 000 à 1 000 000, de 100 000 à 1 000 000, de 10 000 à 100 000. La pluralité de particules permet avantageusement de détecter directement, sans dispositif complexe de visualisation et sans colorant, l'interaction entre lesdites substances au contraire des procédés de l'art antérieur utilisant une seule particule et nécessitant pour la détection de l'interaction des dispositifs complexes de visualisation ou des colorants.

Selon l'invention, lesdites, au moins deux, particules, peuvent être choisies dans le groupe comprenant des particules chargées électriquement, des particules magnétiques, des particules revêtues d'au moins une couche magnétique, des particules magnétisables, des particules revêtues d'une couche magnétisable, des particules électriques, électromagnétiques, électrisables, portant une charge électrique ou un mélange de deux ou plusieurs de ces particules. Il peut s'agir, par exemple de particules constituées entièrement ou en partie d'un matériau magnétique et/ou magnétisable, c'est-à-dire pouvant être mis en mouvement sous l'effet/application d'un champ électrique, magnétique ou électromagnétique. En fait, il peut s'agir de toute particule permettant de mettre en oeuvre la présente invention.

Avantageusement, lesdites particules peuvent être une particule de toute forme adaptée à la mise en oeuvre de la présente invention, par exemple sous forme de bille, de palet, de forme géométrique asymétrique, par exemple avec une face plane, etc.

Toute taille appropriée de particule magnétique peut-être utilisée. La taille peut être choisie par exemple en fonction de la taille du contenant de la solution. Par exemple, la taille des particules peut être inférieure au dixième de la taille du contenant, de préférence inférieure au centième, de manière encore plus préférée inférieur au millième de la taille du contenant. Par exemple la particule peut présenter une taille de, par exemple, 10 nm à 100 µm, de 0,1 à 10 µm.

Avantageusement, le procédé de l'invention permet de mesurer des variations fines des propriétés viscoélastiques de la solution dans laquelle les particules sont immergées. Plus cette variation est faible, plus la mesure doit être sensible. L'invention permet justement et avantageusement en utilisant une pluralité de particules, de détecter des variations faibles causées par des interactions faibles entre des substances qui sont soit en faible quantité, soit mettent en oeuvre des forces d'interactions de faibles intensités. L'utilisation d'une seule particule et/ou bille magnétique ou magnétisable ne permet pas de détecter de manière satisfaisante les interactions. En outre, il existe un risque non négligeable de détection de phénomènes non spécifiques, par exemple causés par présence d'un agrégat, d'impuretés capables d'interférer avec le mouvement de ladite microparticule magnétisable sous l'effet du champ magnétique, électrique, ou électromagnétique.

Au contraire, le procédé de l'invention dans lequel au moins deux particules sont utilisées, permet l'obtention d'un résultat fiable et statistiquement représentatif.

En outre, plus le nombre de particules est important, plus leurs mouvements relatifs apporte d'informations, notamment en permettant une meilleure couverture de la surface au contact de laquelle se développe le phénomène mesuré. Le procédé de l'invention permet donc avantageusement d'interpréter non seulement le mouvement propre desdites microparticules (trajectoire, vitesse de déplacement...), mais aussi l'image globale qu'elles vont dessiner après application du champ magnétique, électrique ou électromagnétique. En effet, la répartition homogène initiale d'une pluralité de microparticules est perturbée par l'application de ce champ en fonction des propriétés viscoélastiques du milieu. Par exemple, si le milieu est très visqueux, par exemple si des interactions fortes entre des substances mises en présence s'y déroulent, la répartition reste homogène, les particules ne pouvant pas ou peu se déplacer sous l'effet dudit champ. Si le milieu est peu ou pas visqueux, ou si des interactions très faibles ou nulles entre lesdites substances mises en présence s'y déroulent, la répartition suivra les lignes de champ les plus importantes, par exemple en formant un anneau ou un spot au dessus d'un aimant cylindrique, les particules ayant toute liberté pour se déplacer sous l'effet dudit champ.

En outre, le procédé de l'invention permet avantageusement, grâce à l'utilisation d'une pluralité de particules, par exemple en répartissant et/ou dispersant lesdites particules de façon homogène dans la solution contenant lesdites substances d'observer visuellement des interactions visibles non seulement à l'échelle moléculaire, c'est-à-dire_de l'ordre du nanomètre, mais aussi à l'échelle microscopique, c'est-à-dire de l'ordre du micron et même macroscopique c'est-à-dire supérieure au micron.

Par exemple des interactions entre des substances conduisant 1) à la formation d'agrégats, de noyaux de nucléation, de cristaux interfèrent de façons différentes avec lesdites particules en comparaison avec des substances dont les interactions conduisent 2) à la formation de polymères plus ou moins réticulés. Dans le premier cas les particules ont tendance à s'accumuler contre les agrégats, noyaux de nucléation, cristaux du coté opposé au sens de mouvement généré par l'application du champ électrique, magnétique ou électromagnétique sur lesdites particules. Les particules génèrent une image contrastée au niveau des zones d'accumulation. Dans le deuxième cas, si la polymérisation se développe de façon homogène et régulière dans la solution, l'image reflète l'immobilisation progressive desdites particules au fur et à mesure de la diminution de l'intensité du champ électrique, magnétique ou électromagnétique (par exemple au fur et à mesure que l'on s'éloigne de l'aimant). Le procédé de l'invention permet donc avantageusement d'obtenir des images révélant les structures découlant des interactions entre les substances et les particules dans la solution.

De préférence, le nombre de particules utilisées pour visualiser des images à l'échelle microscopique et même macroscopique, est compris de 1000 à 1 000 000, de 10 000 à 1 000 000, de 100 000 à 1 000 000, de 10 000 à 100 000.

Selon l'invention les particules peuvent avantageusement avoir une densité proche de la densité des substances susceptibles interagir entres-elles. Par exemple, les particules peuvent avoir, par exemple, une densité relative aux substances comprise en 0,3 et 3.

Selon l'invention, la densité des particules peut être déterminée par tout procédé connu de l'homme du métier, par exemple il peut s'agir du ratio entre la masse de particules et de l'augmentation de volume de la solution dans laquelle sont ajoutées ces particules.

Selon l'invention, la densité des particules permet avantageusement d'améliorer la spécificité et/ou la sensibilité du procédé de l'invention, par exemple en augmentant, en diminuant ou en annulant l'effet de la force de pesanteur.

De préférence, les particules et les substances auront une masse volumique supérieure, à celle de la solution dans lequel elles sont contenues. De préférence, les particules et les substances auront une masse volumique légèrement supérieure, par exemple de 1,0001 à 3 fois supérieure à celle de la solution dans lequel elles sont contenues.

Avantageusement, des molécules d'adhérences peuvent être couplées à la surface des particules. Avantageusement, le couplage desdites molécules à la surface des particules permet l'adhésion des particules entre elles. Par exemple, les particules peuvent être recouvertes de polymères, par exemple de polymères-block comportant une partie hydrophile et une partie hydrophobe, permettant avantageusement après application du champ électrique, magnétique ou électromagnétique un regroupement fixe des particules. En d'autres termes, l'interaction entre les molécules d'adhérences permet de solidariser les particules entres elles après application du champ et donc un résultat stable et non modifiable

Selon l'invention, les particules peuvent être de tailles identiques ou différentes.

Lorsque les particules sont de tailles identiques, les particules sont essentiellement freinées ou accélérées en même temps lors de l'interaction moléculaire. Lorsque les particules utilisées sont de tailles différentes, la taille des petites particules peut être choisie par exemple, de manière à ce qu'elles soient freinées dès le début de l'interaction moléculaire, les grosses particules étant freinée plus tardivement. Dans ce cas, les particules de plus petites tailles sont freinées avant les particules de plus grande taille.

Lorsque les particules sont de tailles différentes, les petites particules peuvent présenter une taille, par exemple, de 10 nm à 1 µm, par exemple de 100 à 500nm, et les grosses particules peuvent présenter une taille, par exemple, de 1 µm à 100µm, par exemple de 1 µm à 10 µm, par exemple de 1 µm à 5 µm.

Avantageusement, des particules de tailles variées peuvent permettre une détection et/ou analyse précoce et plus sensible de l'interaction moléculaire.

Selon l'invention, on peut également utiliser une pluralité de particules de tailles identiques avec une magnétisation différente.

La différence de comportement des différentes particules peut permettre, avantageusement, d'évaluer la force de l'interaction moléculaire. Par l'application du champ magnétique peut provoquer un mouvement des particules ayant été plus fortement magnétisées, la force magnétique est plus grande, tandis que les particules qui ont été plus faiblement magnétisées ne peuvent être mises en mouvement en comparaison avec une immobilisation totales de toute les particules lorsque l'interaction est forte.

Selon l'invention, ladite, au moins une, particule est de préférence une particule génératrice d'un signal détectable. La détection de ce signal sera fonction des propriétés de la particule. À titre d'exemple, ladite, au moins une, particule peut être fluorescente, phosphorescente, radioactive, chimioluminescente, réfléchissante ou colorée.

Par exemple dans le cas où ladite, au moins une, particule est fluorescente, la fluorescence émise par ladite particule peut être détectée par exemple visuellement, et/ou par tous moyens optiques connus de l'homme du métier. Ladite, au moins une, particule peut être par exemple éclairée, pour suivre son mouvement au moyen d'une source lumineuse, par exemple par un faisceau laser.

Selon l'invention, l'éclairage peut être continu ou intermittent. Par exemple l'éclairage peut être réalisé durant toute la durée du procédé ou par exemple durant l'étape a) ou b) ou c), a) et c), a) et b), b) et c).

Par exemple dans le cas où ladite, au moins une, particule est phosphorescente, ladite particule peut être visualisée par exemple visuellement, par tous moyens optiques connue de l'homme du métier.

Par exemple dans le cas ou ladite particule est radioactive, ladite particule peut être détectée même au travers de liquides ou milieux de cultures optiquement opaques, ainsi qu'au travers de plaques de microdilution optiquement opaques par tous dispositifs de détection de la radioactivité émise connue de l'homme du métier, notamment la méthode classique de révélation sur film autoradiographique. Il suffit alors de plaquer le film sensible sous la plaque de microdilution et de révéler ensuite l'image du fond de ladite plaque de microdilution.

Par exemple dans le cas où ladite, au moins une, particule est chimioluminescente, la détection de ladite particule peut se former par ajout dans le milieu du réactif chimique permettant l'émission d'énergie lumineuse par ladite particule. La détection de ce signal peut être par exemple visuelle et/ou par tous moyens connus de l'homme du métier notamment par l'utilisation de caméra CCD (« Charges Coupled Device ») sensible aux longueurs d'ondes émises, qui balaient (scannent) les cupules de la plaque de microdilution.

Par exemple dans le cas où ladite, au moins une, particule est réfléchissante la détection de ladite particule peut être par exemple visuelle ou par tous moyens optiques connue de l'homme du métier. Avantageusement, ladite, au moins une, particule peut être par exemple éclairée, pour suivre son mouvement au moyen d'une source lumineuse, par exemple par un faisceau laser.

Par exemple dans le cas où lesdites, au moins deux, particules sont colorées, la détection desdites particules peut être par exemple visuelle et/ou par tout moyen connue de l'homme du métier pour la détection de particules colorées.

Selon l'invention, lesdites particules peuvent avantageusement être choisies de couleurs différentes en fonction de leur taille et/ou en fonction de leur pouvoir magnétique. L'application du champ magnétique permet dans ce cas l'apparition d'un point coloré ou anneau ou d'une tache colorée sur la surface par regroupement desdites particules. Cette caractéristique permet avantageusement une détection visuelle facilitée du regroupement. En effet, par exemple dans le cas où lesdites particules sont de deux tailles différentes, les grosses particules étant d'une couleur et les petites d'une autre, lors de l'application du champ magnétique si l'interaction entre lesdites substances est faible les petites particules restent immobilisées dans la solution tandis que les grosses particules sont regroupées. Ce regroupement est visible par l'apparition d'un point coloré qui dans ce cas est identique à la couleur des particules de plus grande taille. Si aucune interaction moléculaire n'est présente dans la solution entre les substances, les petites et les grandes particules peuvent êtres regroupés et le rassemblement des particules est visualisé par apparition d'un point coloré correspondant à la superposition des deux couleurs. Enfin si l'interaction moléculaire est importante, les particules peuvent être totalement freinées et ne peuvent donc pas êtres regroupés et aucun point de couleur n'est visible.

En d'autres termes, le regroupement peut être évalué, par exemple, en mesurant la tache formée par lesdites particules regroupée. Par exemple, lorsque lesdites particules sont colorées, en mesurant la couleur desdites particules. En outre, le regroupement peut être évalué, par exemple en fonction de la forme de la tache obtenue, par exemple en fonction de la forme, du diamètre, sa topologie, par exemple un disque, un anneau.

L'homme du métier comprendra aisément que pour la réalisation de la présente invention, le choix des propriétés visuelles desdites, au moins deux, particules peut également se faire en fonction de la solution. En effet la détection du mouvement desdites, au moins deux, particules est d'autant plus facile que le contraste entre la couleur desdites, au moins deux, particules et la couleur de la solution est grand.

Dans la présente invention, le champ magnétique, ou électrique ou électromagnétique peut être tout champ permettant de mettre en mouvement lesdites, au moins deux particules sur ladite surface immergée dans ladite solution, par exemple un champ électromagnétique ou un champ magnétique. Le champ magnétique, ou électrique ou électromagnétique peut être généré, par exemple par un aimant ou par un solénoïde. L'aimant peut être par exemple sous forme de barreau, de pointe, de pièce, etc. ou toute forme appropriée pour la mise en oeuvre de la présente invention. Le champ peut, par exemple être appliqué par tout moyen connu de l'homme du métier, par exemple par impulsion, par augmentation progressive du champ électromagnétique, par variations de champ électromagnétique ou par une combinaison ces applications.

Une augmentation progressive du champ électromagnétique peut être obtenue, par exemple, par rapprochement d'un aimant selon une trajectoire rectiligne ou sinusoïdale, ou selon un mouvement oscillant présentant ou non une amplitude d'oscillation et/ou une fréquence variables. Des variations de champ plus complexes peuvent être obtenues, par exemple par rotation ou par des combinaisons de mouvements d'un matériau aimanté à proximité de ladite, au moins une, particule.

Ainsi, selon l'invention, ledit champ magnétique peut être généré par des moyens générateurs de champ qui peuvent être ou non en mouvement.

Lorsque plusieurs particules doivent être mises en mouvement, le champ doit pouvoir regrouper lesdites particules sur ladite surface immergée dans ladite solution.

Quel que soit le mode de mise en oeuvre de l'invention, le procédé de l'invention peut avantageusement être réalisé simultanément dans une pluralité de compartiments.

Dans ce cas, pour la mise en mouvement des particules dans lesdits compartiments, une pluralité de champs magnétiques, par exemple d'aimants, peuvent avantageusement être utilisés. Les aimants peuvent par exemple êtres fixés sur un support de telle manière à permettre une juxtaposition de chaque compartiment dans lequel on réalise le procédé de l'invention avec un aimant du support. L'application du champ magnétique sur lesdits compartiments est indépendante d'un compartiment à un autre. Le champ magnétique appliqué auxdits compartiment peut être identique ou différent d'un compartiment à l'autre, de préférence identique.

Selon l'invention, le champ magnétique, électromagnétique, électrique peut être appliqué, par exemple pendant un temps de 1 seconde à 15 minutes, de 10 secondes à 10 minutes. Bien entendu, l'homme du métier de par ces connaissances générales saura adapter le temps en fonction des substances testées et/ou de la puissance du champ appliqué.

Par compartiment dans la présente invention, on entend par exemple un réacteur de culture, des cupules, des tubes ou des puits par exemple de plaques de microdilution. On entend par plaque de microdilution le type de plaque défini par exemple par l'American National Standards Institute et la Society for Biomolecular Sciences (« microplates ») portant 96 puits, 384 et même 1536.

Le matériau constituant ledit compartiment peut être tout matériau adapté à la réalisation de la présente invention, par exemple du plastique, par exemple du polycarbonate, du polystyrène, etc., du verre, du métal, etc. Avantageusement, on peut utiliser des plaques de microdilution en polystyrène présentant des cupules sans fonds. Le fond de ces cupules est obtenu en fixant une surface plane sous la plaque de microdilution. Cette surface plane peut être en matériaux transparents, par exemple en plastique, en verre, etc. ou en matériaux opaques, par exemple en métal, en céramique, etc. Le mode de fixation de la surface plane sous la plaque de microdilution peut être tout mode approprié connu de l'homme du métier pour une telle fixation, par exemple par collage au moyen d'une colle, par une méthode par friction, par collage par fusion de la matière plastique, par exemple au laser, etc.

Selon l'invention, les différents compartiments peuvent êtres regroupés sur un même support, par exemple sur une plaque comportant de 1 à 1536 puits, par exemple 6, 16, 64, 96, etc.

Le compartiment peut correspondre par exemple à une enceinte avec une extrémité fermée, du type tube, puits, etc. ou une enceinte présentant deux ouvertures.

Selon une première configuration, le compartiment peut présenter une extrémité fermée de sorte à former un fond plat.

Selon une seconde configuration, le compartiment peut présenter une extrémité fermée de sorte à former un fond hémisphérique.

Selon l'invention, la détermination de l'interaction entre les dites substances peut être effectuée directement après application du champ magnétique, électromagnétique, électrique. Par exemple la détermination peut être effectuée de 0,01 seconde à 30 minutes, de 1 seconde à 3 minutes après application du champ.

Selon l'invention, la détermination de l'interaction entre lesdites substances peut être également réalisée en comparant le rassemblement des particules lors de l'application du champ magnétique, électromagnétique, électrique. Par exemple, il est possible de comparer le rassemblement des particules dans une solution ne contenant aucune ou une seule desdites substances précitées avec le rassemblement des particules dans une solution comprenant ladite première et ladite seconde substance. Si le rassemblement est plus important ou s'il est moins important dans la solution ne comprenant aucune ou une seule desdites substances alors lesdites substances interagissent et cette interaction est ainsi déterminée. En d'autres termes, l'interaction entre les substances peut diminuer ou augmenter le rassemblement des particules lors de l'application dudit champ et peut être ainsi déterminée. Par exemple, si l'interaction provoque une agrégation des substances, le milieu peut devenir moins visqueux et le rassemblement de particules sera augmenté. Par exemple si l'interaction provoque une précipitation des substances, la surface peut être, par exemple encombrée par le précipitât et le rassemblement des particules sera diminué. Par exemple, si l'interaction provoque une réticulation des substances entre elle, le réseau de substances va ralentir les particules et le rassemblement de particules sera diminué. Par exemple, si l'interaction provoque la formation de chaînes linéaires de substances, alors la viscosité du milieu sera augmentée et le rassemblement des particules sera diminué.

Selon l'invention, le procédé peut-être réalisé par exemple dans une pluralité de compartiments comprenant chacun :
- ladite solution,
- ladite, au moins une particule, et
- lesdites substances susceptibles d'interagir entre elles

Selon l'invention, le procédé peut comprendre en outre une étape b' de mélange. Le mélange peut être réalisé, par exemple par agitation de la solution dans laquelle sont présentes lesdites substances. Il peut s'agir par exemple, d'un mélange avec un agitateur magnétique, d'un mélange avec un agitateur orbital. Selon l'invention, le mélange peut être réalisé, par exemple pendant 1 seconde à 15 minutes, de 10 secondes à 10 minutes.

La présente invention a également pour objet l'utilisation du procédé de l'invention pour, par exemple, l'identification de substances présentes dans une solution, l'analyse d'échantillons biologiques, la réalisation de tests biologiques, la réalisation de typage sanguin, la réalisation de tests d'immuno-hématologie, la réalisation de détection de contaminations naturelles ou d'origine humaine, par exemple par des virus, par des bactéries, par des amibes, par des levures, par des cellules cancéreuses, par des prions, par des pesticides, par des fongicides, par des antibiotiques, par des hormones, par des toxines. La présente invention a également pour objet l'utilisation du procédé de l'invention pour, par exemple, la réalisation de test de dopages, la réalisation d'études scientifiques sur des substances, par exemple pour étudier l'effet de solution sur l'interaction entre des substances, par exemple dans des solutions aqueuses, dans des solutions organiques, dans des gaz, par exemple avec des molécules, des colloïdes, des nanoparticules, des microparticules. La présente invention a également pour objet l'utilisation du procédé de l'invention pour, par exemple, la réalisation d'études scientifiques sur des sujets ou des substances sont impliquées, par exemple en biologie, en cancérologie, en endocrinologie, en infectiologie, par exemple pour l'étude des hydrogels formés dans des solutions aqueuses, des aérogels formés dans des gaz, des gels formés dans des solvants ou dans des hydrocarbures.

Par exemple, le procédé de l'invention peut être utilisé pour l'analyse d'échantillons biologiques, par exemple pour le sérotypage sanguin, la détermination du CMH d'un individu, la détermination de la présence, par exemple, d'une protéine, d'un anticorps, d'un récepteur particulier dans un échantillon. Le procédé de l'invention peut également être utilisé pour l'identification de substance chimique dans une solution.

Par exemple, lorsque le procédé de l'invention est utilisé pour le sérotypage, la solution est un échantillon de sang provenant d'un individu, par exemple d'un être humain ou d'un animal, comprenant la première substance.

En outre, le procédé de l'invention peut être utilisé pour la détermination de l'interaction entre un récepteur et un ligand éventuel, entre un anticorps et une protéine, une molécule chimique, un autre anticorps et/ou toute molécule susceptible d'être reconnue par un anticorps. Le procédé de l'invention peut être également utilisé pour détecter l'interaction entre des cellules, par exemple entre des cellules eucaryotes, des cellules procaryotes, entre des cellules eucaryotes et procaryotes. Le procédé de l'invention peut être également utilisé pour détecter indépendamment l'interaction entre des bactéries, des virus, des levures et/ou des cellules eucaryotes.

Le procédé de l'invention peut être également utilisé, par exemple pour détecter des phénomènes de chimiotaxie, des phénomènes d'adhésion cellulaire.

Le procédé de l'invention peut-être également, par exemple, utilisé pour détecter l'interaction entre des nanoparticules, entre des microparticules, entre des objets de forme complexe de taille microscopique et nanoscopique.

Le procédé de l'invention peut également, par exemple, être utilisé pour détecter l'interaction (i) d'une première substance, par exemple des anticorps produits par un humain ou un animal en réponse à une infection, par exemple par un virus, par une bactérie, par un prion, par un parasite, par un protozoaire, mais aussi en réponse à une maladie, par exemple un cancer, une maladie auto-immune comme par exemple la maladie de Basedow, la sclérose en plaque, mais aussi en réponse à une intoxication, à un empoisonnement, à une contamination, à un dopage, à une ingestion, à une inhalation et à une injection, par exemple par un pesticide, par un poison, par un insecticide, par un herbicide, par un agent allergisant, mais aussi en réponse à une greffe, par exemple de moelle osseuse, d'organe comme par exemple un rein, un poumon, un foie, de membre comme par exemple de main, de jambe, de pied, mais aussi en réponse à une implantation d'organe artificiel, de chambre implantable, de pacemaker, de coeur artificiel, de hanche artificielle, et (ii) d'une seconde substance, par exemple une substance susceptible d'avoir provoqué la production de ladite première substance, par exemple un antigène responsable de la production desdits anticorps précités.

Selon l'invention, la solution peut comprendre la seconde substance susceptible d'avoir provoqué la production de ladite première substance à détecter.

Avantageusement, la seconde substance peut être fixée à la surface du contenant, la fixation de ladite seconde substance permet avantageusement de détecter une interaction entre lesdites première et seconde substances quelque soit leurs concentrations, par exemple entre des anticorps et des antigènes même lorsque ceux-ci sont présents en faible quantité.

Selon l'invention, le procédé de l'invention peut comprendre en outre l'utilisation (iii) d'une troisième substance.

Il peut s'agir par exemple d'une substance telle que définie précédemment, par exemple des anticorps susceptibles de reconnaitre au moins une desdites première et/ou seconde substance. Il peut s'agir par exemple d'anticorps idiotypiques, par exemple des anticorps reconnaissant spécifiquement des anticorps produits par un être humain ou un animal, par exemple des anticorps anti-lapin, des anticorps anti-chèvre, des anticorps anti IgG, ou anti IgM, ou anti IgA, ou anti IgE.

Selon l'invention, la troisième substance peut être préalablement fixée à la surface du contenant permettant, par exemple la détection de l'interaction de ladite troisième substance avec ladite première et/ou seconde substance.

Avantageusement, selon l'invention la troisième substance est un anticorps idiotypique, c'est-à-dire un anticorps anti anticorps qui peut interagir par exemple avec un anticorps produit par un humain ou un animal en réponse à un antigène et/ou un antigène séjournant ou ayant séjournée dans un homme ou un animal comme indiqué précédemment.

La présente invention permet donc avantageusement de détecter indirectement une substance, par exemple une substance séjournant ou ayant séjournée dans un homme ou un animal sans avoir besoin de développer un anticorps interagissant spécifiquement avec cette substance.

La présente invention permet donc avantageusement de détecter directement ou indirectement la présence d'anticorps produits en réponse à infection, une maladie, une intoxication, un empoisonnement, une contamination, un dopage, une ingestion, un pesticide, une greffe. En outre, la présente invention permet donc de détecter des infections silencieuses, c'est-à-dire par exemples des infections non détectables par les moyens microbiologiques communs, par exemple les hémocultures, les cultures de biopsies, de liquides physiologiques prélevés baignant ou irrigant le site présumé d'infection, par exemple du liquide céphalorachidien, de l'urine, de la salive, en détectant, par exemple en utilisant des anticorps idiotypiques interagissant avec des anticorps dirigés spécifiquement contre ces infections.

D'autres avantages pourront encore apparaître à l'homme du métier à la lecture des exemples ci-dessous, illustrés par les figures annexées, donnés à titre illustratif.

### Brève description des figures

- La figure 1 représente des photographies des tâches obtenues avec comme substance d'affinité des anticorps présentant (sw H) ou ne présentant pas (sw E) d'affinité entre eux. La photographie 1A a été prise après 20 secondes d'aimantation, la photographie 1B après 5 minutes d'aimantation.
- La figure 2 représente des photographies des tâches obtenues avec comme substance d'affinité des anticorps anti déterminant de groupe sanguin et des hématies.
- La figure 3 représente des images des tâches obtenues avec comme substance d'affinité des anticorps anti déterminant de groupe sanguin et des hématies.

### EXEMPLES

### Exemple 1 : Détection de l'interaction d'anticorps présentant ou ne présentant pas d'affinité entre eux.

Dans cet exemple, deux barrettes de 8 puits à fond plat (Référence : MSW002B, BioFilm Control, France), notées, respectivement, swH, swE ont été utilisées. Dans chacune des barrettes, il a été déposé dans chaque puits 70 µl de solution d'anticorps obtenue en mélangeant 15 µl de respectivement soit des anticorps anti IgG humaines (Référence BI2018, Paris Anticorps, France), soit des anticorps anti E.coli (Référence BP2298, Acris Antibodies, Allemagne), dans 1,5 ml de tampon PBS (8g/l NaCl (Sigma Aldrich, USA), 200mg/l KCI (Sigma Aldrich, USA)), 1.44 g/l Na₂HPO₄ (Sigma Aldrich, USA), 240mg/l KH₂PO₄ (Sigma Aldrich, USA)) avant de les placer pendant 16 heures dans une étuve thermostatique (Référence BC240, Firelabo, France) stabilisée à 37°C.

Des solutions notées respectivement, Slg0, SlgH et SlgE, contenant 200 µl de réactif de recherche d'anticorps humains anti-érythrocytaires anti-FY1 (IJB-IH Contrôle 3, référence 108030357, Institut Jacques Boy, France), 2 µl de microbilles paramagnétiques (Ton004N, BiofilmControl, France) et respectivement soit 15 µl d'eau pour préparation injectable, soit 200µl d'anticorps anti IgG humaines (Référence BI2018, Paris Anticorps, France), soit 200µl d'anticorps anti E.coli (Référence BP2298, Acris Antibodies GmbH, Allemagne) ont été préparées.

Les solutions d'anticorps ont été ensuite retirées des barrettes swH et swE, puis 150µl de tampon PBS ont été déposées et retirées par trois fois dans chaque puits.

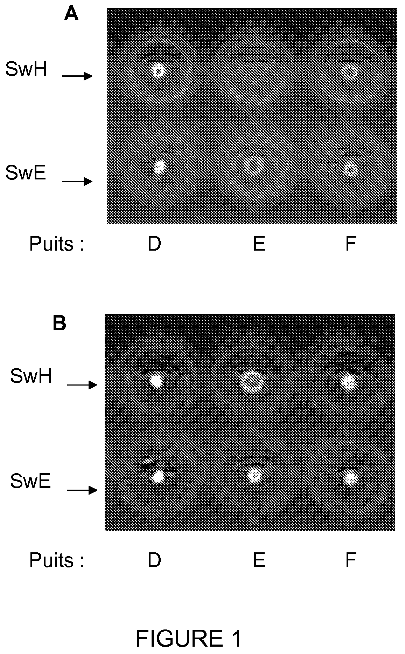

Il a été déposé respectivement dans les puits, D, E et F des barrettes swH et swE, 100 µl de Slg0, SlgH et SlgE. Les barrettes ont été ensuite placées pendant 20 minutes dans une étuve thermostatique (Référence BC240, Firelabo, France) stabilisée à 37°C, puis placées sur un bloc test aimanté (BKT-MSW002 BioFilm Control, France) pendant 20 secondes. Les barrettes ont été ensuite placées sur un scanner de documents (perfection V-750 PRO, Epson, USA) avec lequel une première prise d'image a été effectuée avec le logiciel EpsonScan (Epson, USA). Le cycle aimantation/prise d'image a été reproduit une seconde fois afin de disposer également d'une seconde image après un total de 5 minutes d'aimantation. Les images finales ont été obtenues en retranchant la composante verte des images de la composante rouge des images couleurs obtenues avec le scanner en utilisant le logiciel ImageJ (http://rsb.info.nih.gov.ij) et un découpage des images obtenues avec différents réglages du contraste. Les photographies correspondantes correspondent aux images de la figure 1.

Des anneaux de différents diamètres ont été obtenus, correspondant à l'accumulation de billes au fond du puits au cours de leur migration dans le champ magnétique. Le diamètre de l'anneau est considéré comme une mesure selon une loi décroissante de la mobilité magnétique des billes.

Comme attendu, le diamètre de l'anneau observé en présence de la réaction d'affinité attendue entre les immuno-globulines présentes dans le réactif de recherche d'anticorps anti-érythrocytaires et les anticorps anti-Ig Humaines (barrette swH puits E) est plus important qu'en absence de réaction d'affinité entre les immuno-globulines présentes dans réactif de recherche d'anticorps anti-érythrocytaires et les anticorps anti-E.coli (barrette swH puits D et F) après 20 secondes de magnétisation, et dans les puits E, après 5 minutes de magnétisation (barrettes swH et swE puits E).

Comme visible sur la figure 1, puits E, cet effet est amplifié lorsque les deux substances d'affinité sont présentes simultanément en solution et adsorbée dans le puits, ce qui réduit fortement la migration des billes (barrette swH puits E) par rapport à la situation, représente sur la figure 1, barrette swH puits D où l'une des substances n'est présente qu'adsorbée sur la surface.

En outre, lorsque les deux substances d'affinité, à savoir les anticorps, sont présentes en solution, si une des substances est déjà adsorbée sur la surface, le phénomène de réduction de la mobilité des billes est encore amplifié (puits E, barrette SwH par rapport à SwE).

Enfin, lorsque la substance d'affinité n'est pas adsorbées sur la surface, la réalisation de la réaction d'affinité dans la solution est détectable en observant le diamètre de l'anneau formé (barrette SwE, puits E par rapport au puits D et F).

### Exemple 2 : détection de l'interaction entre anticorps anti déterminant de type de groupe sanguin et d'hématies.

Dans cet exemple, trois barrettes de 8 puits au format SBS à fond plat (Référence : MSW002B, BioFilm Control, France), notées, respectivement, swA, swB et swAB ont été utilisées. Dans chacun des puits des barrettes 70 µl de solution d'anticorps, respectivement, anti A (Référence 102010153, Institut Jacques Boy, France), anti B (102010253, Institut Jacques Boy, France) et anti AB (Référence 102010353, Institut Jacques Boy, France) ont été déposées.

Les barrettes ont été placées dans une étuve thermostatique (Référence BC240, Firelabo, France) stabilisée à 37°C pendant 10 minutes.

Des suspensions sanguines notées, respectivement, hA,hB,hAB et h0, ont été préparées en diluant 1 ml de tampon TS composé de 8g/l NaCl (Sigma-Aldrich, USA) et de 1 g/l de tryptone (Difco, USA) avec, respectivement, 100 µl d'hématies de type respectivement A, B, AB, et 0 (IJB-IH Contrôle 2, Référence 108020257, Institut Jacques Boy, France) et 6 µl de microbilles paramagnétiques (Ton005N, BiofilmControl, France) et 1 µl de colorant alimentaire bleu (Vahiné, France).

Les solutions d'anticorps ont été retirées des puits avant de déposer 70 µl de préparation respectivement, hA, hB, hC et h0, dans les puits respectivement, A et B, puis C et D, puis E et F, puis G et H des barrettes swA, swB et swAB.

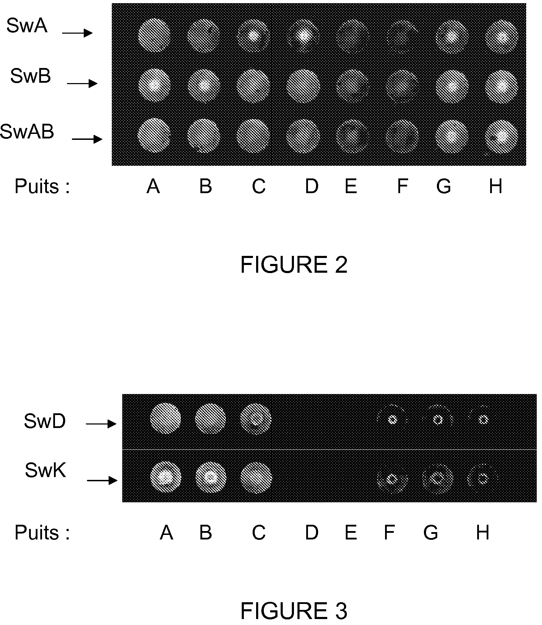

Les barrettes ont été placées dans une étuve thermostatique (Référence BC240, Firelabo, France) stabilisée à 37°C pendant 10 minutes puis placées sur le bloc test aimanté (BKT-MSW002 BioFilm Control, France) pendant 1 minute. Elles ont été ensuite placées sur un scanner de documents (perfection V-750 PRO, Epson, USA) avec lequel une prise d'image a été effectuée avec le logiciel EpsonScan (Epson, USA). L'image finale a été obtenue comme dans l'exemple 1 dans la figure 2.

Comme montré sur la figure 2 dans les puits C, D, G et H de la barrette SwA, dans les puits A, B, G et H de la barrette SwB et dans les puits G et H de la barrette SwAB, il a été observé la formation d'un disque sombre d'un diamètre d'environ 2 mm +/- 1 mm qui est attribué à une absence de réaction d'affinité entre les anticorps liés au puits et les hématies. Dans les autres puits aucune forme sombre n'est visible, démontrant une réaction d'affinité entre les anticorps liés au puits et les hématies. Les observations sont résumées dans le tableau 1 suivant, où l'absence de disque est notée - et la présence de disque est notée + :

**Tableau 1 : observation des puits de la figure 2**

| | A (hA) | B (hA) | C (hB) | D (hB) | E (hAB) | F (hAB) | G (h0) | H (h0) |
|---|---|---|---|---|---|---|---|---|
| SwA (anti A) | - | - | + | + | - | - | + | + |
| SwB (anti B) | + | + | - | - | - | - | + | + |
| SwAB(anti AB) | - | - | - | - | - | - | + | + |
| Typage déduit | A | A | B | B | AB | AB | 0 | 0 |

Comme démontré dans cet exemple, les résultats obtenus montrent donc clairement la détection de l'afifinité des anticorps avec les hématies correspondantes. Par ailleurs, cet exemple démontre clairement que le procédé de l'invention permet de déterminer le sérotype d'un échantillon de sang.

### Exemple 3 : détection de l'interaction entre des anticorps anti déterminant d'anticorps anti-érythrocytaires.

Dans cet exemple deux barrettes de 8 puits à fond plat (Référence : MSW002B, BioFilm Control, France), notées swD et swK ont été utilisées. Dans chacun des puits des barrettes, 50 µl d'une solution obtenue en mélangeant 15 µl d'anticorps anti IgG humaine (Référence BI2018, Paris Anticorps, France) dans 1,5 ml de tampon TS (8 g/l NaCl(Sigma Aldrich, USA), 1g/l Tryptone (Difco, USA)) a été déposée. Les barrettes ont été ensuite incubées pendant 16 heures dans une étuve thermostatique (Référence BC240, Firelabo, France) stabilisée à 37°C.

Des solutions notées respectivement, Sp et St, comportant 150 µl de tampon, respectivement PBS et TS, et 0,75 µl de Ton005N ont été préparées. Ces solutions correspondent aux solutions témoins sans hématies.

Des suspensions notées, respectivement, Si, Sii et Siii, comportant 150 µl d'une suspension d'hématies de type I, II et III (Référence ID-DiaCell I-II-III, Diamed, France) et 0,75 µl de microbilles paramagnétiques (Ton005N, BiofilmControl, France) ont été également préparées.

Les antigènes présents à la surface des hématies ont été vérifiés par le fournisseur et se répartissent selon le tableau 2 suivant :

**Tableau 2 :**

| Suspensions | Hématies | Duffy | KEL |
|---|---|---|---|
| Si | Hématies type I | + | 0 |
| Sii | Hématies type II | + | 0 |
| Siii | Hématies type III | 0 | + |

| | | | |
|---|---|---|---|
| + : présence de l'antigène, 0 : absence de l'antigène. | | | |

Des suspensions notées, respectivement, SDi et SKi, SDii et SKii ainsi que SDiii et SKiii, comportant 100 µl d'une suspension d'hématies de type respectivement I, II et III (Référence ID-DiaCell I-II-III, Diamed, France), 50 µl de sérum, respectivement anti FY1 (ou anti Duffy) et anti KEL1 (IJB-IH Contrôle 3, référence 108030357, Institut Jacques Boy, France) et 0,75 µl de microbilles paramagnétiques (Ton005N, BiofilmControl, France) ont été également préparées (voir tableau 3). Ces suspensions sont des contrôles négatifs comportant des hématies dont les sites d'affinités sont bloqués par les anticorps contenus dans les sérums, ainsi les complexes hématie-anticorps ne présentent plus de sites d'affinités libres pouvant se lier aux anticorps fixés au fond du puits.

**Tableau 3 :**

| | Hématies | Serum |
|---|---|---|
| SDi | I (D) | Anti FY1 (anti D) |
| SKi | I (D) | Anti KEL1 (anti K) |
| SDii | II (D) | Anti FY1 (anti D) |
| SKii | II (D) | Anti KEL1 (anti K) |
| SDiii | III (K) | Anti FY1 (anti D) |
| SKiii | III (K) | Anti KEL1 (anti K) |

La solution d'anticorps anti IgG humaines a été ensuite retirée des barrettes swD et swK et 150µl de tampon TS a déposé et retiré par deux fois. Dans chaque puits des barrettes swD et swK a été déposé respectivement, 50 µl de réactifs de recherche d'anticorps anti-érythrocytaires, respectivement anti-FY1 (ou anti Duffy) et antiKEL1 (IJB-IH Contrôle 3, référence 108030357, Institut Jacques Boy, France). Les barrettes ont été ensuite placées pendant 10 minutes dans une étuve thermostatique (Référence BC240, Firelabo, France) stabilisée à 37°C.

Les solutions de réactifs de recherche d'anticorps anti-érythrocytaires ont été ensuite retirées des barrettes swD et swK et 150µl de tampon TS a été déposés et retirés par deux fois.

70 µl des différentes suspensions ont été déposés comme indiqué dans le tableau 4 ci-dessous :

**Tableau 4 : répartition du dépôt des différentes suspensions**

| | A | B | C | D | E | F | G | H |
|---|---|---|---|---|---|---|---|---|
| swD | Si | Sii | Siii | Sp | St | SDi | SDii | SDiii |
| swK | Si | Sii | Siii | Sp | St | SKi | SKii | SKiii |

Les barrettes ont été ensuite placées pendant 20 minutes dans une étuve thermostatique (Référence BC240, Firelabo, France) stabilisée à 37°C, puis placées sur le bloc test aimanté (BKT-MSW002 BioFilm Control, France) pendant 30 secondes. Les barrettes ont été ensuite placées sur un scanner de documents (perfection V-750 PRO, Epson, USA) avec lequel une prise d'image est effectuée avec le logiciel EpsonScan (Epson, USA). Cette image sert à l'analyse de la barrette swK. Le cycle aimantation/prise d'image a été reproduit une seconde fois afin de disposer également d'une image après un total d'une minute d'aimantation servant à l'analyse de la barrette swD. Les images finales sont obtenues comme dans l'exemple 1 et sont représentées dans la figure 3.

La figure 3 montre qu'un anneau parfaitement défini est visible dans les puits contrôles D et E, témoignant d'une mobilité importante des billes paramagnétique, un anneau similaire est obtenu dans les puits F, G et H, témoignant qu'une mobilité importante est conservée en présence d'hématies dont les sites d'affinités sont bloqués par les anticorps contenus dans les sérums.

Dans les puits A, B et C, on peut vérifier la présence ;
- soit d'un anneau comparable à ceux des puits F, G et H, témoignant qu'une mobilité importante attribuée à une absence de lien d'affinité entre les hématies et les anticorps liés au fond du puits,
- soit d'une tâche diffuse témoignant d'une mobilité réduite des billes paramagnétiques attribuées à une réaction d'affinité entre les anticorps liés au puits et les hématies.

Anneaux et tâches sont visibles selon le tableau 5 suivant :

**Tableau 5 : images obtenues**

| | A | B | C | D | E | F | G | H |
|---|---|---|---|---|---|---|---|---|
| swD | tâche | tâche | anneau | anneau | anneau | anneau | anneau | anneau |
| swK | anneau | anneau | tâche | anneau | anneau | anneau | anneau | anneau |

Le procédé de l'invention permet donc de détecter l'interaction moléculaire entre deux substances.

### Exemple 4 : détection de l'interaction entre des anticorps anti bactériens présents dans un sérum et des substances issues de cultures de bactéries

Dans cet exemple, deux barrettes de 8 puits à fond plat (Référence : MSW002B, BioFilm Control, France), notées, respectivement, swH, swE sont utilisées. Dans chacune des barrettes, on dépose dans chaque puits 70 µl de solution d'anticorps obtenue en mélangeant 15 µl de respectivement, anticorps anti IgG humaines (Référence BI2018, Paris Anticorps, France) et anticorps anti E.coli (Référence BP2298, Acris Antibodies, Allemagne), dans 1,5 ml de tampon PBS (8g/l NaCl (Sigma Aldrich, USA), 200mg/l KCI (Sigma Aldrich, USA)), 1.44 g/l Na₂HPO₄ (Sigma Aldrich, USA), 240mg/l KH₂PO₄ (Sigma Aldrich, USA)) avant de les placer pendant 16 heures dans une étuve thermostatique (Référence BC240, Firelabo, France) stabilisée à 37°C (voir tableau 6).

**Tableau 6 :**

| | A | B | C | D | E | F | G | H |
|---|---|---|---|---|---|---|---|---|
| swH | Anti Humain | Anti Humain | Anti Humain | Anti Humain | Anti Humain | Anti Humain | Anti Humain | Anti Humain |
| swE | anti | anti | anti | anti | anti | anti | anti | anti |
| | E.coli | E.coli | E.coli | E.coli | E.coli | E.coli | E.coli | E.coli |

La solution d'IgG est ensuite retirée des barrettes swH et swE et 150µl de tampon TS est déposé et retiré par deux fois. Dans chaque puits, respectivement A, B, C, D, et E, F, G, H, des barrettes swH et swE est déposé 150 µl de sérum humain provenant, respectivement, de patients souffrant d'une infection à staphylocoque (sérum Staph +) et de témoins ne souffrant pas d'une telle infection (sérum Staph -) (voir tableau 7). Les barrettes sont ensuite placées pendant 10 minutes dans une étuve thermostatique (Référence BC240, Firelabo, France) stabilisée à 37°C.

**Tableau 7 :**

| | A | B | C | D | E | F | G | H |
|---|---|---|---|---|---|---|---|---|
| swH | Serum Staph + / Anti Humain | Serum Staph + / Anti Humain | Serum Staph + / Anti Humain | Serum Staph + / Anti Humain | Serum Staph - / Anti Humain | Serum Staph - / Anti Humain | Serum Staph - / Anti Humain | Serum Staph - / Anti Humain |
| swE | Serum Staph + / anti E.coli | Serum Staph + / anti E.coli | Serum Staph + / anti E.coli | Serum Staph + / anti E.coli | Serum Staph - / anti E.coli | Serum Staph - / anti E.coli | Serum Staph - / anti E.coli | Serum Staph - / anti E.coli |

50 µl de réactif de détection d'infection à staphylocoque comprenant des substances issues de cultures de staphylocoque et des microbilles paramagnétiques (Référence: RDS-01 R, BioFilm Control, France), sont déposés dans les puits des barrettes.

Les barrettes sont ensuite placées pendant 20 minutes dans une étuve thermostatique (Référence BC240, Firelabo, France) stabilisée à 37°C, puis placées sur le bloc test aimanté (BKT-MSW002 BioFilm Control, France) pendant 10 secondes. Les barrettes sont ensuite placées sur un scanner de documents (perfection V-750 PRO, Epson, USA) avec lequel une prise d'image est effectuée avec le logiciel EpsonScan (Epson, USA). Le cycle aimantation/prise d'image est reproduit une seconde fois afin de disposer également d'une image après un total de 5 minutes d'aimantation. Les images finales sont obtenues comme dans l'exemple 1.

Le diamètre de l'anneau observé en présence de la réaction d'affinité attendue entre les immuno-globulines présentes dans le sérum de patient infecté et les substances contenues dans le réactif de détection de staphylocoque (puits A, B, C et D) est plus important qu'en absence de réaction d'affinité dans le sérum de patients non infecté (puits E, F, G et H)

Listes des références
1. US 3 635 678 CLOT-TIMING SYSTEM AND METHOD, Seitz, L.J. and Bowen, J.G. ;
2. WO 01/86255, METHOD AND APPARATUS FOR DETERMINING LOCAL VISCOELASTICITY, Cronin-Golomb, M. Shabtai, Y. Nemet, B.;
3. EP1544596, Méthode et appareil de détermination de la viscosité, Kurowski D.; Schoen C.; Peters R.; Bartos H.; Yu Y. ;
4. Catterall WA. (2000) Structure and regulation of voltage-gated Ca2+ channels. Annu. Rev. Cell. Dev. Biol. 16:521-55
5. Gerald Karp, Biologie cellulaire et moléculaire, Concepts et expériences, 2nd édition 2004
6. Dick GM and Tune JD, Role of potassium channels in coronary vasodilation, Exp Biol Med (Maywood). 2010 Jan;235(1):10-22
7. Mostallino MC et al. Plasticity and function of extrasynaptic GABA(A) receptors during pregnancy and after delivery, Psychoneuroendocrinology. 2009 Dec;34 Suppl 1:S74-83

## Revendications

1. Procédé de détection d'interactions dans une solution comprenant les étapes suivantes:
a. introduire dans une solution au moins une première substance et au moins une seconde substance susceptible d'interagir avec ladite première substance,
b. introduire dans la solution obtenue à l'étape a) de 2 à 10 000 000 particules magnétiques ou magnétisables lesdites particules reposant sur une surface immergée dans ladite solution, lesdites particules ayant une taille comprise de 10 nm à 100 µm,
c. déterminer l'interaction desdites substances par application d'un champ électrique, magnétique ou électromagnétique destiné à mettre en mouvement lesdites particules, l'interaction entre lesdites substances étant détectée lorsque la mobilité desdites particules sur ladite surface est modifiée.

2. Procédé selon la revendication 1, dans lequel lesdites particules magnétiques ou magnétisables sont indépendamment une particule chargée électriquement, magnétique ou magnétisable ou recouverte d'au moins une couche magnétique ou magnétisable.

3. Procédé selon l'une quelconque des revendications 1 à 2, dans lequel lesdites particules sont soumises à un champ électromagnétique appliqué par impulsion.

4. Procédé selon l'une quelconque des revendications 1 à 3, dans lequel la modification de la mobilité est une accélération du mouvement des particules sous l'effet dudit champ électrique, magnétique ou électromagnétique.

5. Procédé selon l'une quelconque des revendications 1 à 3, dans lequel la modification de la mobilité est un ralentissement du mouvement des particules sous l'effet dudit champ électrique, magnétique ou électromagnétique.

6. Procédé selon l'une quelconque des revendications 1 à 3, dans lequel la modification de la mobilité est une modification de la trajectoire des particules sous l'effet dudit champ électrique, magnétique ou électromagnétique.

7. Procédé selon l'une quelconque des revendications 1 à 3, dans lequel l'interaction est détectée par le rassemblement des particules sous l'effet dudit champ électrique, magnétique ou électromagnétique.

8. Procédé selon l'une quelconque des revendications 1 à 3, dans lequel l'interaction est détectée par le non-rassemblement des particules sous l'effet dudit champ électrique, magnétique ou électromagnétique.

9. Procédé selon l'une quelconque des revendications 1 à 3, dans lequel l'interaction est détectée par la dispersion des particules sous l'effet dudit champ électrique, magnétique ou électromagnétique.

10. Procédé selon l'une quelconque des revendications précédentes, dans lequel lesdites, au moins deux, particules sont éclairées au moyen d'une source lumineuse pour détecter son mouvement.

11. Procédé selon l'une quelconque des revendications précédentes, dans lequel lesdites particules, au moins deux, sont génératrices d'un signal.

12. Procédé selon l'une quelconque des revendications 1 à 11 dans lequel la première substance est choisie dans le groupe comprenant des cellules eucaryotes, des cellules procaryotes, des membranes, des virus, des protéines, des anticorps, des antigènes, des molécules chimiques.

13. Procédé selon l'une quelconque des revendications 1 à 11 dans lequel la seconde substance est choisie dans le groupe comprenant des cellules eucaryotes, des cellules procaryotes, des membranes, des virus, des protéines, des anticorps, des antigènes, des molécules chimiques.

14. Procédé selon l'une quelconque des revendications précédentes dans lequel le procédé comprend à la place de l'étape a)
une étape préalable a') de fixation de ladite première substance sur une surface d'un contenant,
a") l'introduction d'une solution dans ledit contenant, et
a"') l'introduction d'au moins une seconde substance susceptible d'interagir avec ladite première substance.

## Patentansprüche

1. Verfahren zum Nachweis von Wechselwirkungen in einer Lösung, umfassend die folgenden Schritte:
a. Einbringen von mindestens einer ersten Substanz und mindestens einer zweiten Substanz, die mit der ersten Substanz wechselwirken kann, in eine Lösung,
b. Einbringen von 2 bis 10 000 000 magnetischen oder magnetisierbaren Partikel in die in Schritt a) erhaltene Lösung, wobei diese Partikel auf einer in die Lösung eingetauchten Oberfläche aufliegen, wobei die Partikel eine Größe von 10 nm bis 100 µm besitzen,
c. Bestimmen der Wechselwirkung der Substanzen durch Anlegen eines elektrischen, magnetischen oder elektromagnetischen Felds mit dem Ziel, die Partikel in Bewegung zu setzen, wobei die Wechselwirkung zwischen den Substanzen nachgewiesen wird, wenn die Mobilität der Partikel auf der Oberfläche modifiziert ist.

2. Verfahren nach Anspruch 1, wobei die magnetischen oder magnetisierbaren Partikel unabhängig ein elektrisch geladenes, magnetisches oder magnetisierbares Partikel oder ein mit mindestens einer magnetischen oder magnetisierbaren Schicht bedecktes Partikel sind.

3. Verfahren nach einem der Ansprüche 1 bis 2, wobei die Partikel einem durch Impuls angelegten elektromagnetischen Feld unterworfen werden.

4. Verfahren nach einem der Ansprüche 1 bis 3, wobei es sich bei der Modifikation der Mobilität um eine Beschleunigung der Bewegung der Teilchen unter der Wirkung des elektrischen, magnetischen oder elektromagnetischen Felds handelt.

5. Verfahren nach einem der Ansprüche 1 bis 3, wobei es sich bei der Modifikation der Mobilität um eine Verlangsamung der Bewegung der Teilchen unter der Wirkung des elektrischen, magnetischen oder elektromagnetischen Felds handelt.

6. Verfahren nach einem der Ansprüche 1 bis 3, wobei es sich bei der Modifikation der Mobilität um eine Modifikation der Bahn der Teilchen unter der Wirkung des elektrischen, magnetischen oder elektromagnetischen Felds handelt.

7. Verfahren nach einem der Ansprüche 1 bis 3, wobei die Wechselwirkung durch das Zusammenkommen der Teilchen unter der Wirkung des elektrischen, magnetischen oder elektromagnetischen Felds nachgewiesen wird.

8. Verfahren nach einem der Ansprüche 1 bis 3, wobei die Wechselwirkung durch das Nicht-Zusammenkommen der Teilchen unter der Wirkung des elektrischen, magnetischen oder elektromagnetischen Felds nachgewiesen wird.

9. Verfahren nach einem der Ansprüche 1 bis 3, wobei die Wechselwirkung durch die Zerstreuung der Teilchen unter der Wirkung des elektrischen, magnetischen oder elektromagnetischen Felds nachgewiesen wird.

10. Verfahren nach einem der vorhergehenden Ansprüche, wobei die mindestens zwei Partikel mithilfe einer Lichtquelle belichtet werden, um ihre Bewegung nachzuweisen.

11. Verfahren nach einem der vorhergehenden Ansprüche, wobei die mindestens zwei Partikel ein Signal erzeugen.

12. Verfahren nach einem der Ansprüche 1 bis 11, wobei die erste Substanz aus der Gruppe ausgewählt ist, die eukaryotische Zellen, prokaryotische Zellen, Membranen, Viren, Proteine, Antikörper, Antigene, chemische Moleküle umfasst.

13. Verfahren nach einem der Ansprüche 1 bis 11, wobei die zweite Substanz aus der Gruppe ausgewählt ist, die eukaryotische Zellen, prokaryotische Zellen, Membranen, Viren, Proteine, Antikörper, Antigene, chemische Moleküle umfasst.

14. Verfahren nach einem der vorhergehenden Ansprüche, wobei das Verfahren anstelle von Schritt a)
einen Schritt a') der Fixierung der ersten Substanz auf einer Oberfläche eines Behälters,
a") des Einbringens einer Lösung in den Behälter und
a"') des Einbringens von mindestens einer zweiten Substanz, die mit der ersten Substanz wechselwirken kann, umfasst.

## Claims

1. A method for detecting interactions in a solution comprising the following steps:
a. introducing into a solution at least one first substance and preferably at least one second substance that can interact with said first substance,
b. introducing into the solution obtained in step a) at least 2 to 10 000 000 magnetic or magnetizable particles, said particles resting on a surface immersed in said solution, said particles having a size of 10 nm to 100 µm,
c. determining the interaction of said substances by application of an electric, magnetic or electromagnetic field designed to set said particles in motion, the interaction between said substances being detected when the mobility of said particles on said surface is changed.

2. The method according to claim 1, wherein said magnetic or magnetizable particles are independently an electrically charged, magnetic or magnetizable particle or a particle covered with at least one magnetic or magnetizable layer.

3. The method according to any of claims 1 or 2, wherein said particles are subjected to a pulsed electromagnetic field.

4. The method according to any one of claims 1 to 3, wherein the mobility shift is an acceleration of the movement of particles under the effect of said electric, magnetic or electromagnetic field.

5. The method according to any one of claims 1 to 3, wherein the mobility shift is a slowing of the movement of the particles under the effect of said electric, magnetic or electromagnetic field.

6. The method according to any one of claims 1 to 3, wherein the mobility shift is a change in trajectory of the particles under the effect of said electric, magnetic or electromagnetic field.

7. The method according to any one of claims 1 to 3, wherein the interaction is detected by clustering of the particles under the effect of said electric, magnetic or electromagnetic field.

8. The method according to any one of claims 1 to 3, wherein the interaction is detected by non-clustering of the particles under the effect of said electric, magnetic or electromagnetic field.

9. The method according to any one of claims 1 to 3, wherein the interaction is detected by dispersion of the particles under the effect of said electric, magnetic or electromagnetic field.

10. The method according to any one of the preceding claims, wherein said at least two particles are illuminated by means of a luminous source to detect their movement.

11. The method according to any one of the preceding claims, wherein said at least two particles are signal generators.

12. The method according to any one of claims 1 to 11, wherein the first substance is chosen in the group comprising eukaryotic cells, prokaryotic cells, membranes, viruses, proteins, antibodies, antigens, chemical molecules.

13. The method according to any one of claims 1 to 11, wherein the second substance is chosen in the group comprising eukaryotic cells, prokaryotic cells, membranes, viruses, proteins, antibodies, antigens, chemical molecules.

14. The method according to any one of the preceding claims, wherein the method includes, instead of step a),
a prior step a') of attaching said first substance onto a surface of a container,
a") introducing a solution into said container, and
a'") introducing at least one second substance that can interact with said first substance.
